# EUROPÄISCHE PATENTSCHRIFT

(11) **EP 1 960 330 B1**
(45) Veröffentlichungstag und Bekanntmachung des Hinweises auf die Patenterteilung: **02.11.2011**
(21) Anmeldenummer: 06828601.2
(22) Anmeldetag: 04.12.2006
(51) Int. Cl.: C07C 13/615, C07C 23/46

(54) **VERFAHREN ZUR HERSTELLUNG VON SUBSTITUIERTEN DIAMANTANEN**
METHOD FOR PRODUCING SUBSTITUTED DIAMANTANES
PROCEDE POUR PREPARER DES DIAMANTANES SUBSTITUES

(30) Priorität: 06.12.2005 DE 102005058357
(43) Veröffentlichungstag der Anmeldung: 27.08.2008
(73) Patentinhaber: Justus-Liebig-Universität Gießen, 35390 Giessen (DE)
(72) Erfinder: SCHREINER, Peter R., Prof. Dr., 35435 Wettenberg (DE); FOKIN, Andrey, A., 35398 Giessen (DE)
(74) Vertreter: Buchhold, Jürgen
(86) Internationale Anmeldenummer: PCT/DE2006/002146
(87) Internationale Veröffentlichungsnummer: WO 2007/065409

(56) Entgegenhaltungen:
- OLAH G A ET AL: "NITRATION OF ADAMANTANE AND DIAMANTANE WITH NITRONIUM TETRAFLUOROBORATE" JOURNAL OF THE AMERICAN CHEMICAL SOCIETY, AMERICAN CHEMICAL SOCIETY, WASHINGTON, DC, US, Bd. 115, Nr. 16, 11. August 1993 (1993-08-11), Seiten 7246-7249, XP000383576 ISSN: 0002-7863
- BLANEY F ET AL: "Diamondoid rearrangements in chlorosulphonic acid. A highly regioselective route to apically disubstituted diamantanes" TETRAHEDRON LETTERS, ELSEVIER, AMSTERDAM, NL, Nr. 2, 1975, Seiten 99-100, XP003006712 ISSN: 0040-4039
- ANDREY A FOKIN ET AL: "Functionalized nanodiamonds part I. An experimental assessment of diamantane and computational predictions for higher diamondoids" CHEMISTRY - A EUROPEAN JOURNAL, VCH PUBLISHERS, US, Bd. 11, Nr. 23, 2005, Seiten 7091-7101, XP009086802 ISSN: 0947-6539
- A.FOKIN ET AL: "Reactivity of (1(2,3)4)Pentamantane" J.ORG.CHEM., Bd. 71, 2006, Seiten 8532-8540, XP009086971
- M.C.DAVIS ET AL: "Preparation of Diamines of Adamantane and Diamantane from the Diazides" SYNTHETIC COMMUNICATIONS, Bd. 36, 2006, Seiten 2113-2119, XP009087004

## Beschreibung

Die vorliegende Erfindung beschreibt Verfahren zur selektiven Herstellung von derivatisierten Diamantanen, wobei die Bindung von schwer trennbaren Produktgemischein vermieden wird und Ausbeuten von mindestens 60%, bezogen auf das als Edukt eingesetzte Diamantan, erhalten werden.

Diamantane sind als Ausgangsstoffe für die Mikroelektronik, die Pharmazie, die Nanotechnologie und die Materialwissenschaften von Interesse. Potenzielle Anwendungen sind beispielsweise die Herstellung temperaturstabiler Kunststoffe, Beschichtungen mit maßgeschneiderten Leitfähigkeiten für LEDs und Transistoren, die Nanoelektronik sowie die Verwendung in Pharmazeutika gegen virale und neurodegenerative Erkrankungen.
Von besonderem Interesse sind hierbei Diamantane mit Hydroxy-, Carbonyl-, Carboxyh Amino- und/oder Aminocarbonylgruppen, da sie wichtige Zwischenprodukte für die Herstellung oligomerer und polymerer Diamantane sind. Außerdem lassen sich die genannten funktionellen Gruppen durch andere funktionelle Gruppen substituieren, so dass auf diese Weise substituierte Diamantane wichtige Ausgangsstoffe für die Herstellung weiterer substituierter Diamantane darstellen.

Der Stand der Technik kennt einige Verfahren zur Herstellung hydroxylierter Diamantane. Die bisher bekannten Verfahren liefern jedoch nur geringe Ausbeuten an hydroxylierten Diamantanen und/oder erfordern aufwändige Reinigungsschritte (beispielsweise Chromatographie) und/oder verwenden erfordern die Verwendung von Feinchemikalien, d.h. hoch reinen Lösungsmitteln und Reagenzien.

So ergibt die Oxidation von Diamantan 1 in 96% Schwefelsäure nur 5% des entsprechenden Dihydroxyderivats 2, neben den Hauptprodukten 3 und 4 (Courtney, T.; Johnston, D. E.; McKervey, M. A.; Rooney, J. J. Chemistry of diamantane. 1. Synthesis and functionalization reactions. Journal of the Chemical Society, Perkin Transactions 1: Organic and Bio-Organic Chemistry (1972-1999) (1972), (21), 2691-6). Bei dieser Methode ist die Ausbeute des gewünsähten Produktes 2 sehr schlecht, und die entstehende Mischung ist nur mit hohem Aufwand zu trennen. 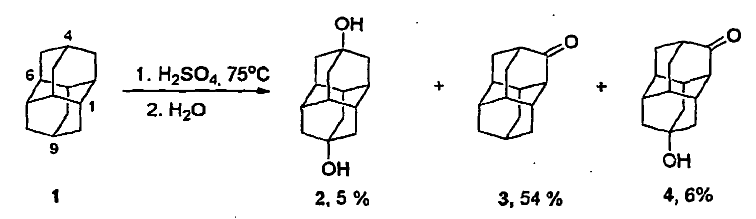 Reduktion des Dimers von Norbornadien (Binor-S, 5) zu einer Mischung von Tetrahydro-Derivaten 6, anschließende Reaktion mit reiner Chlorosulfonsäure und Hydrolyse des Dichlorids 7 in Gegenwart von NaOH im Autoklaven ergibt ebenfalls das 4,9-Dihydroxydiamantan 2. (Blaney, Frank; Johnston, Don E.; McKervey, M. Anthony; Rooney, John J. Diamondoid rearrangements in chlorosulfonic acid. Highly regioselective route to apically disubstituted diamantanes. Tetrahedron Letters (1975), (2), 99-100). Nachteile dieser Methode sind die Verwendung eines über mehrere Stufen hergestellten Ausgangsmaterials und die Notwendigkeit einer Autoklavenreaktion. Diamantan ist in sehr großen Mengen direkt aus Erdöl zugänglich. Deshalb sind Methoden, die 1 als Ausgangsmaterial zur Darstellung von. 3 nutzen, bevorzugt.

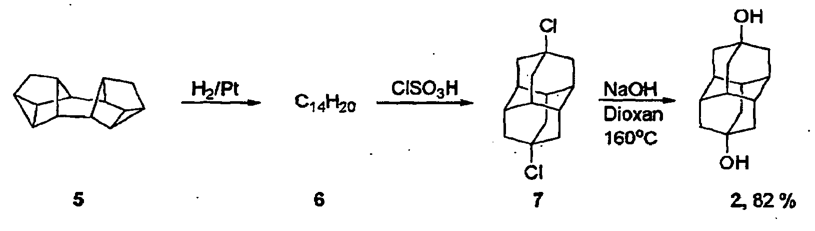

Oxidation von 4-Hydroxydiamantan 8 mit dem Pilz *Rhizopus nigricans* ergibt eine Mischung von 1,9-Dihydroxydiamantan 9 und 4,9-Dihydroxydiamantan 2 in einem Verhältnis von 5:1 in 69% Ausbeute (Blaney, Frank; Johnston, Don E.; McKervey, M. Anthony; Jones, Ewart R. H.; Pragnell, John. Hydroxylation of diamantan-1-and -4-oi with the fungus Rhizopus nigricans. Journal of the Chemical Society, Chemical Communications (1974), (8), 297-8). Nachteil dieser Methode ist, dass 7 als sehr teures Ausgangsmaterial eingesetzt wird und eine schwer trennbare Mischung entsteht, in der 2 nur zu etwa 15% enthalten ist

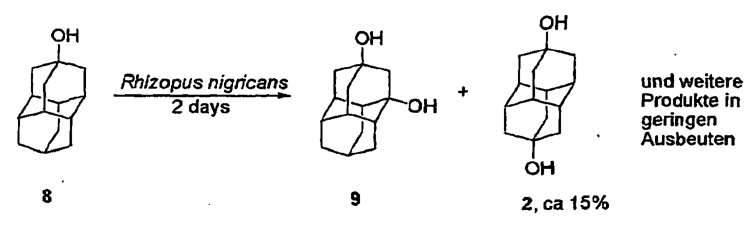

Bromierung von Diamantan zu 4,9-0ibromodiamantan 10, gefolgt von einer Hydrolyse zu 2 (Janku, Josef; Burkhard, Jiri; Vodicka, Ludek. Hydrolysis of bromine derivatives of diamantane with nitric acid. Zeitschrift fuer Chemie (1981), 21(9), 325-6). Diese Methode verläuft nur mit geringer Selektivität bei der Bromierung und dementsprechend geringer Ausbeute an 3.

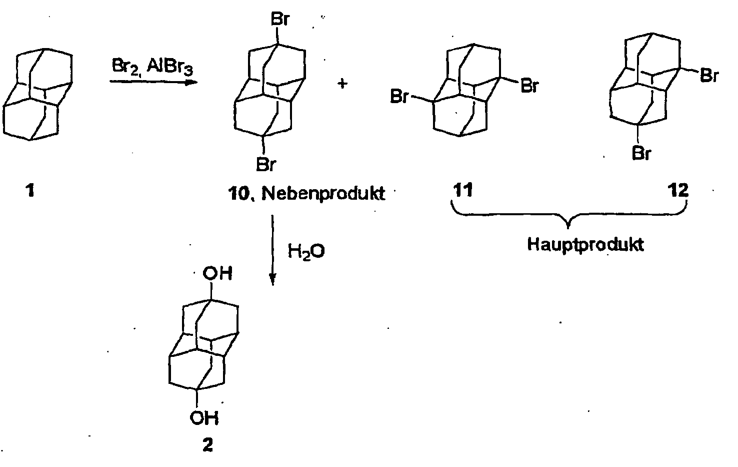

Die US 2005/0074690 A1 beschreibt Polymere enthaltend Monomereinheiten aus Diamantanen. In dieser In dieser Schrift wird ein Verfahren zur Herstellung von zweifach bis vierfach hydroxylierten Diamantanen durch Umsetzung von Diamantan mit N Hydroxyphthalimid und Co-II-acetylacetonat beschrieben. Nachteilig ist, dass ein Produktgemisch entsteht und die Ausbeute gering ist.
Die US 5,430,193 und die US 5,410,092 beschreiben Verfahren zur Überführung eines Diarnantan-Lactons in das korrespondierende Hydroxyketon, wobei Hydroxy- und Carbonylgruppe durch mindestens ein Brückenkopf-GAtom getrennt sind. Dabei wird das Diamantoid-Lacton mit einem Anhydrid in Gegenwart von. Säure umgesetzt und der als Zwischenprodukt entstehende Ketoester hydrolysiert. Die selektive Einführung funktioneller Gruppen in unsubstituierte Diamantane ist mit diesen Verfahren jedoch nicht möglich.

Die WO 03/050066 A1 und die WO 021057201 A2 beschreiben funktionalisierte bzw. polymerisierbare höhere Diamantane. Allerdings geben beide Schriften keine Hinweise auf die Herstellung der derivatisierten Diamantane.

Die Oxidation von Diamantan 1 mit *m*-Chlorperbenzoesäure oder HNO₃ als Oxidationsmittel und CH₂Cl₂ als Lösungsmittel ist in AA Fokin, BA Tkachenko, PA Guchenko, DV Gusev, PR Schreiner: Functionalized Nanodiamonds Part I. An Experimental Assessment of Diamantane and Computational Predictions for Higher Diamondoids. Chem. Eur. J. 2005, 11, 7091-7101 beschrieben. Allerdings entsteht bei Verwendung -von Salpetersäure und anschließender Umsetzung mit Wasser ein Gemisch verschiedener Monohydroxydiamantane (8 und 13); bei Verwendung von m-Chlorperbenzoesäure entstehen sogar Gemische verschiedener Mono- und Dihydroxyadarnantane.

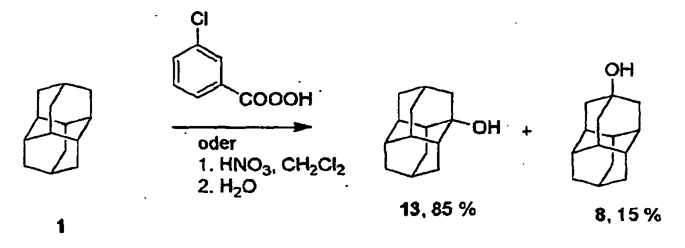

Die vorliegende Erfindung zeichnet sich gegenüber dem Stand der Technik dadurch aus, dass sie es gestattet, substituierte Diamantane aus den korrespondierenden nitroxylierten Diamantanen selektiv herzustellen.
Überraschend wurde gefunden, dass
- mononitroxylierte Diamantane sich mit zahlreichen Nucleophilen abfangen lassen und somit als Zwischenprodukte für die Herstellung einfach substituierter Diamantane geeignet sind, und dass
- die dem Fachmann bisher nicht bekannten mindestens zweifach nitroxylierten Diamantane zur selektiven Herstellung mindestens zweifach derivatisierter Diamantane verwendet werden können.
Die vorliegende Erfindung stellt somit neue, bisher nicht bekannte mindestens zweifach substituierte Diamantane bereit.

Des Weiteren liefert die vorliegende Erfindung Verfahren, um substituierte Diamantane herzustellen. Dabei werden die als Edukt verwendeten Diamantane in einem ersten Schritt nitroxyliert. Die Nitroxygruppe ist eine gute Abgangsgruppe und lässt sich daher gut durch andere Nucleophile substituieren. In einem weiteren Schritt werden die nitroxylierten Diamantane mit Nucleophilen umgesetzt

Überraschend wurde gefunden, dass im Falle einer mindestens zweifachen Nitroxylierung mindestens eine Nitroxygruppe an ein apicales Kohlenstoffatom des Diamantans gebunden wird.
Des Weiteren wurde im Rahmen der Arbeiten, die zur vorliegenden Erfindungen führten, gefunden, dass sich zweifach nitroxylierte sowie zweifach hydroxylierten Diamantane in Gegenwart einer starken Säure umlagern, wenn eine der beiden Nitroxy- oder Hydroxygruppen zuvor an ein apicales Kohlenstoffatom gebunden war. Bei dieser Umlagerung wandert eine Nitroxy- oder Hydroxygruppe, der zuvor nicht an ein apicales Kohlenstoffatom gebunden war, an ein apicales Kohlenstoffatom, an das zuvor Wasserstoff gebunden war. Das Umlagerungsprodukt kann anschließend mit weiteren Nucleophilen umgesetzt werden.

Dem Fachmann ist bekannt, wie er Hydroxygruppen in andere funktionelle Gruppen überführen kann. So lassen sich Hydroxyverbindungen durch Umsetzung mit Ameisensäure in konzentrierter Schwefelsäure zu den korrespondierenden Carbonsäuren umsetzen. Diese Koch-Haaf-Reaktion ist beispielsweise in L Vodicka, J Janku, J Burkhard: "Synthesis of diamantanedicarboxylic acid with the carboxy groups bonded at tertiary carbon atoms." Lab. Synth. Fuels, Prague Inst. Chem.-Technol., Prague, Czech. Collection of the Czechoslovak Chemical Communications (1983), 48 (4), 1162-1172 beschrieben. Dem Fachmann ist ebenfalls bekannt, dass er die Koch-Haaf-Reaktion auch verwenden kann, um nitroxylierte Verbindungen in die entsprechenden Carbonsäuren zu überführen. Diese Reaktion ist in TM Gund, M Nomura, PvR Schleyer, J. Org. Chem. 1974, 39, 2987-2994, beschrieben.
Die Einführung von Alkylaminogruppen ist dem Fachmann bekannt und beispielsweise in AA Fokin, BA Tkachenko, PA Guchenko, DV Gusev, PR Schreiner Functionalized Nanodiamonds Part I. An Experimental Assessment of Diamantane and Computational Predictions for Higher Diamondoids. Chem. Eur. J. 2005, 11, 7091-7101 am Beispiel der Umsetzung von bromierten Diamantanen mit Acetonitril beschrieben. Der Fachmann weiß, dass er in analoger Weise auch Hydroxy- und Nitroxygruppen in Alkylaminogruppen überführen kann.
Dem Fachmann ist bekannt, wie er Hydroxy- und Nitroxygruppen in Alkylcarbonsäureester überführen kann, wobei OH- bzw. -ONO₂-Gruppen durch OCO-AlkylGruppen substituiert werden. So ist die Herstellung von 1-Acetoxydiamantan aus 1-Hydroxydiamantan und Essigsäure beispielsweise in J Janku, L Vodicka, J Jeziorsky. Sbornik Vysoke Skoly Chemicko-Technologicke v Praze, D: Technologie Paliv. 1984, D49, 5-23 beschrieben.

### Aufgabe der Erfindung

Aufgabe der Erfindung ist es, mindestens zweifach nitroxylierte Diamantane sowie ein Verfahren zu deren Herstellung bereitzustellen. Eine weitere Aufgabe der Erfindung ist es, die Verfahren zur Herstellung substituierter Diamantane zu verbessem, wobei unter Verbesserung eine Erhöhung der Ausbeute und/oder eine Erhöhung der Selektivität der Produktbildung verstanden wird.

### Lösung der Aufgabe

Die erfindungsgemäßen, mindestens zweifach nitroxylierten Diamantane besitzen die allgemeine Strukturformel 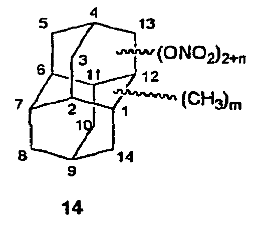
wobei n eine ganze Zahl zwischen 0 und 6 darstellt,
m eine ganze Zahl zwischen 0 und 1 darstellt,
die Methylgruppe im Falle von m = 1 bevorzugt an Position 1, 3 oder 4 des Diamantans gebunden ist,
und mindestens eine Nitroxygruppe an ein apicales Kohlenstoffatom gebunden ist.

Die Aufgabe, verbesserte Verfahren zur Herstellung der substituierter Diamantane bereitzustellen, wird erfindungsgemäß gelöst, indem in einem ersten Schritt 1 Äquivalent des Diamantans mit je 1,8 bis 20,0 Äquivalenten eines Nitroxylierungsmittels pro einzuführender Nitroxygruppe umgesetzt wird. Optional findet die Umsetzung in einem organischen Lösungsmittel statt Bevorzugt wird das organische Lösungsmittel ausgewählt aus der Gruppe CH₂Cl₂, CHCl₃, CCl₄, Diethylether, THF, Ethylacetat oder Gemischen davon.

Erfindungsgemäß werden die nitroxylierten Diamantane in einem weiteren Schritt mit einem Nucleophil umgesetzt, wobei das Nucleophil alle Nitroxygruppen substituiert.

Überraschend wurde gefunden, dass Ausbeute und/oder Selektivität der Herstellung mindestens zweifach substituierter Diamantane signifikant gesteigert werden, indem Diamantane **1** zunächst mindestens zweifach nitroxyliert werden und das dabei entstehende Produktgemisch anschließend in Gegenwart eines Nucleophils zum korrespondierenden Gemisch mehrfach substituierter Diamantoide umgesetzt wird. Dabei bezieht sich der Begriff substituiert auf solche funktionellen Gruppen, die erst mit Hilfe der erfindungsgemäßen Verfahren eingeführt werden oder eingeführt worden sind.
Es wurde gefunden, dass bei der mindestens zweifachen Nitroxylierung von Diamantanen mindestens eine Nitroxylgruppe in einer apicalen Position des Diamantans gebunden wird, d.h. in Position 4 oder 9. Die Nitroxygruppe ist eine gute Abgangsgruppe und lässt sich durch andere nucleophile Gruppen ersetzen. Dem Fachmann ist bekannt, wie er diese Nucleophile einführen muss. Ferner ist bekannt, dass und wie die entstehende Produktgemische getrennt und gereinigt werden können, beispielsweise durch Extraktion, Umkristallisation oder Chromatographie.

In einer bevorzugten Ausführungsform der vorliegenden Erfindung wird das mindestens zweifach nitroxylierte Diamantan in Gegenwart einer starken Säure umgelagert. Überraschend wurde gefunden, dass sich mindestens zweifach nitroxylierte Diamantane, bei denen mindestens eine Nitroxygruppe an ein apicales Kohlenstoffatom gebunden ist, bei Zugabe einer starken Säure umlagern. Bei dieser Umlagerung wandert eine Nitroxygruppe, die zuvor nicht an ein apicales Kohlenstoffatom gebunden war, an ein apicales Kohlenstoffatom, an das zuvor Wasserstoff gebunden war. Alternativ können die mindestens zweifach nitroxylierten Diamantane, die mindestens eine apicale Nitroxygruppe tragen, zunächst mit Wasser (als Nucleophil) hydrolysiert werden und anschließend in Gegenwart einer starken Säure umgelagert werden. Im Rahmen der vorliegenden Erfindung wurde festgestellt, dass sich nicht nur Dinitroxy-, sondern auch Dihydroxydiamantane in Gegenwart einer starken Säure umlagern, wenn zuvor mindestens eine apicale Nitroxy- oder Dihydroxygruppe an ein apicales Kohlenstoffatom gebunden war. Nitrierte Verbindungen sind dagegen nicht in der Lage, die oben beschriebene Umlagerungsreaktion einzugehen. Dem Fachmann ist bekannt, dass es nach dem derzeitigen Stand der Technik keine Möglichkeit gibt, die Nitrogruppe -NO₂ in eine Nitroxygruppe -ONO₂ überzuführen. Die erfindungsgemäße mindestens zweifache Nitroxylierung ist daher der entscheidende Schritt bei der Herstellung mindestens zweifach substituierter Diamantane mit hoher Ausbeute und/oder Selektivität.

Die saure Umlagerung von mindestens zweifach nitroxylierten Diamantanen ist beispielhaft, aber nicht erschöpfend, für 1,4-Dinitroxydiamantan **16** gezeigt:

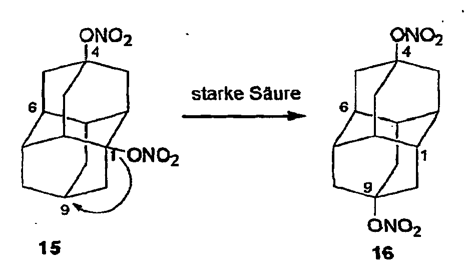

Die saure Umlagerung von mindestens zweifach hydroxylierten Diamantanen ist beispielhaft, aber nicht erschöpfend, für 1,4-Dihydroxydiamantan **2** gezeigt:

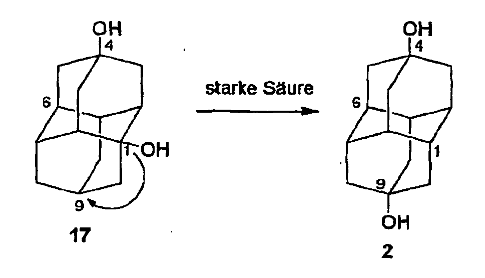

Dem Fachmann stehen hiermit folgende erfindungsgemäße Verfahren zur Herstellung mindestens zweifach substituierter Diamantane zur Verfügung:
a) mindestens zweifache Nitroxylierung, gefolgt von der Substitution aller Nitroxygruppen mit einem Nucleophil oder
b) mindestens zweifache Nitroxylierung, anschließende Umlagerung in Gegenwart einer starken Säure, nach erfolgter Umlagerung werden alle Nitroxygruppen durch ein Nucleophil ersetzt oder
c) mindestens zweifache Nitroxylierung, anschließende Umsetzung mit Wasser (als Nucleophil), Umlagerung der mindestens zweifach hydroxylierten Verbindung in Gegenwart einer starken Säure, nach erfolgter Umlagerung werden alle Hydroxygruppen durch ein weiteres Nucleophil ersetzt.

Die erfindungsgemäßen Verfahren zur Herstellung mindestens zweifach substituierter Diamantoide sind nachfolgend beschrieben.

### 1. Herstellung von mindestens zweifach nitroxytierten Diamantoiden

Die Herstellung der mindestens zweifach nitroxylierten Diamantoide nach dem erfindungsgemäßen Verfahren wird nach folgender allgemeiner Vorschrift durchgeführt 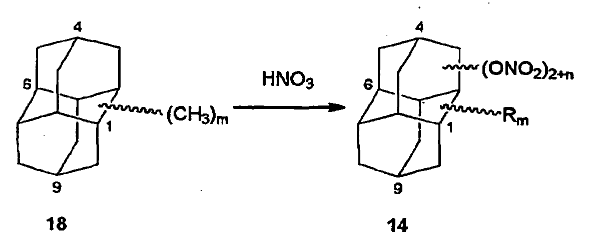 Dabei sind m, n und R wie auf S. 8 beschrieben definiert.
1 Mol des Diamantoids **18** wird mit 0 bis 2 Litern eines unpolaren organischen Lösungsmittels und 8 Mol bis 25 Mol eines Nitroxylierungsreagenzes pro einzuführender Nitroxygruppe bei -5 °C bis 5°C gemischt und für 1 h bis 3 h bei 20°C bis 30°C gerührt.
Das Rohprodukt **14** kann direkt weiter verarbeitet oder optional isoliert werden.

Im letzteren Fall wird überschüssige Säure mit festem NaHCO₃ abgefangen. Der dabei gebildete Niederschlag wird abfiltriert und das unpolare organische Lösungsmittel bei vermindertem Druck entfernt.

Diamantane im Sinne der vorliegenden Erfindung sind beispielsweise Diamantan und dessen einfach oder mehrfach alkylsubstituierten Derivate.
Bevorzugt wird bei der Nitrierung ein organisches Lösungsmittel verwendet, ausgewählt aus der Gruppe halogenierter Kohlenwasserstoff mit ein bis zwei C-Atomen, bevorzugt Methylenchlorid, Chloroform, Tetrachlorkohlenstoff, Diethylether, Tetrahydrofuran, Ethylacetat oder ein Gemisch davon.
Unter "Nitroxylierungsmittel" wird im Rahmen der vorliegenden Erfindung konzentrierte Salpetersäure mit w (HNO₃) = 75% bis 100% oder eine Vorstufe der konzentrierten Salpetersäure verstanden. Vorstufen der konzentrierten Salpetersäure sind beispielsweise Gemische aus konzentrierter Schwefelsäure mit w (H₂SO₄) ≧ 96% und mindestens einem Alkalimetallnitrat, beispielsweise Lithiumnitrat, Natriumnitrat, Kaliumnitrat, oder Gemische aus Ozon und Stickstoffdioxid oder NO⁺X⁻ und NO₂⁺X⁻ Reagenzien, wobei X ein Halogen ist, ausgewählt aus Fluor, Chlor, Brom, Iod. Dem Fachmann ist bekannt, wie er aus diesen Vorstufen der konzentrierten Salpetersäure die konzentrierte Salpetersäure gewinnt.

### 2. Umlagerung von mindestens dinitroxylierten Diamantanen und anschließende Umsetzung mit Wasser

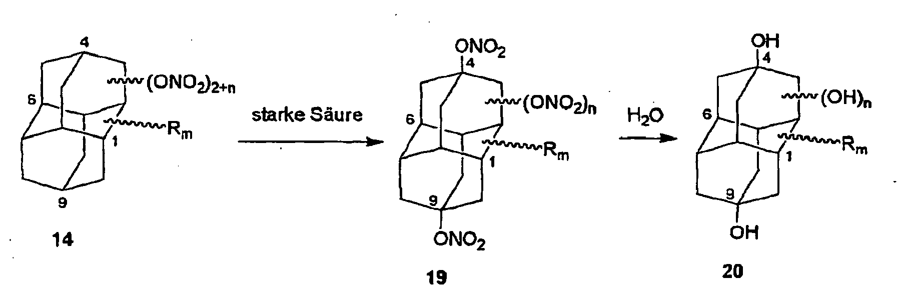

Das Rohprodukt **14** wird zu einer konzentrierten starken Säure gegeben, wobei pro Nitroxygruppe des als Edukt eingesetzten Rohproduktes **14** je 1,5 Mol bis 5 Mol Säure eingesetzt werden. Es wird 1 h bis 3 h bei 20 °C bis 30 °C gerührt. Danach wird die Reaktionsmischung **19** auf Eis gegeben (1 bis 3 Volumenteile Eis pro Volumenteil Reaktionsmischung) und optional filtriert.
Anschließend wird das Reaktionsprodukt **20** gereinigt, wobei die Reinigung mittels Chromatographie, Umkristallisation, diskontinuierlicher oder kontinuierlicher Extraktion erfolgt.

### 3. Umsetzung von mindestens dinitroxylierten Diamantanen mit Wasser und anschließende Umlagerung

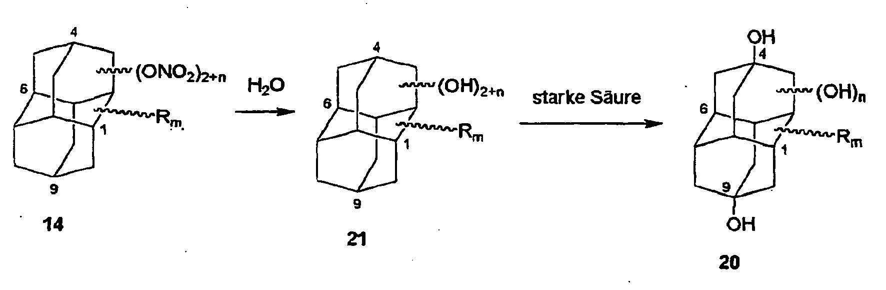

Das Rohprodukt **14** wird nach dem Rühren bei 20°C bis 30 °C mit Wasse r im Verhältnis Reaktionsmischung : Wasser = 1 : 0,3 bis 1: 0,7 verdünnt. Das organische Lösungsmittel wird entfernt und die verbleibende wässrige Phase für 1 h bis 2 h zum Rückfluss erhitzt. Dann wird die Reaktionsmischung auf 20 °C bis 30 °C abgekühlt und filtriert. Der Rückstand bestehend aus einem Gemisch verschiedener hydroxylierter Diamantane **21** wird zu einer konzentrierten starken Säure gegeben, wobei pro Nitroxygruppe des als Edukt eingesetzten Rohproduktes **14** je 1,5 Mol bis 5 Mol Säure eingesetzt werden. Es wird 1 h bis 3 h bei 20 °C bis 30 °C gerührt. Danach wird die Reaktionsmischung **20** auf Eis gegeben (1 bis 2 Volumenteile Eis pro Volumenteil Reaktionsmischung) und optional filtriert.
Optional kann auf die Umlagerung in Gegenwart einer konzentrierten starken Säure verzichtet werden.
Anschließend wird das Reaktionsprodukt gereinigt, wobei die Reinigung mittels Chromatographie, Umkristallisation, diskontinuierlicher oder kontinuierlicher Extraktion erfolgt.

Dem Fachmann ist bekannt, dass starke Säuren solche Säuren sind, deren pKₐ-Wert kleiner oder gleich 2 ist. Für die Umlagerungsreaktion geeignete starke Säuren sind beispielsweise, aber nicht erschöpfend, Schwefelsäure, Salzsäure, Bromwasserstoffsäure, lodwasserstoffsäure, Salpetersäure, Phosphorsäure und Trifluoressigsäure. Bevorzugt wird die starke Säure in konzentrierter Form eingesetzt, wobei unter "konzentriert" solche Lösungen starker Säuren verstanden werden, deren pH-Wert kleiner oder gleich Null ist.
Die optionale Filtration nach der Umlagerung erfolgt bevorzugt über eine Glas- oder Porzellanfritte.
Die optionale Reinigung des Produktes erfolgt durch Umkristallisation, chromatographie oder Extraktion.
Im Falle der Umkristallisation wird ein Lösungsmittel verwendet, das ausgewählt ist aus der Gruppe aliphatische kettenförmige oder cyclische Ether mit zwei bis vierzehn C-Atomen, halogenierte Kohlenwasserstoffe mit ein bis zwei C-Atomen, aliphatische kettenförmige oder cyclische Kohlenwasserstoffe mit vier bis vierzehn C-Atomen, aromatische oder araliphatische Kohlenwasserstoffe mit sechs bis vierzehn C-Atomen und ihren Gemischen. Erfolgt die Reinigung mittels Extraktion, so wird als Extraktionsmittel mindestens ein organisches Lösungsmittel verwendet, ausgewählt aus der Gruppe aliphatische kettenförmige oder cyclische Ether mit zwei bis vierzehn C-Atomen, halogenierte Kohlenwasserstoffe mit ein bis zwei C-Atomen, aliphatische kettenförmige oder cyclische Kohlenwasserstoffe mit vier bis vierzehn C-Atomen, aromatische oder araliphatische Kohlenwasserstoffe mit sechs bis vierzehn C-Atomen und ihren Gemischen.

Dem Fachmann ist bekannt, wie er mittels nucleophiler Substitution ONO₂- oder OH-Gruppen in andere funktionelle Gruppen überführen kann. Beispielhaft, aber nicht erschöpfend, sind hier exemplarisch -NHCOCH₃, -COOH, -NHCOOH als funktionelle Gruppen und CH₃COOH, HCOOH, CH₃CN, H₂O, HCONH₂ als Nucleophile genannt. Wasser selbst dient im Rahmen der vorliegenden Erfindung des Weiteren auch als Nucleophil, mit dem die -ONO₂-Gruppen substituiert werden können.

Diamantanderivate finden Anwendungen beispielsweise in der Pharmakologie, der Mikroelektronik und den Materialwissenschaften. In der Pharmakologie können sie für Pharmakophoren-basiertes Wirkstoffdesign, kombinatorische Drug Discovery, Drug Delivery und für Diagnostika verwendet werden. In der Mikroelektronik sind Diamantoide geeignete Werkstoffe für starre, haltbare elektronische Bauteile im Nanometerbereich, für Anwendungen im Bereich der Feldemission, für Sensoren, für die kontrollierte Einführung von Dotierungssubstanzen wie N, P, B in der Halbleitertechnik sowie als Wachsturnskeim in der Produktion künstlicher Diamanten. Des Weiteren finden Diamantoide Verwendung in den Materialwissenschaften, beispielsweise für Oberflächenfilme, Beschichtungen und Polymere.

Dem Fachmann ist ohne Weiteres ersichtlich, dass sich die in der vorliegenden Erfindung beschriebene Reaktionsfolge aus mindestens zweifacher Nitroxylierung und anschließender Umlagerung auch für die Herstellung anderer Diamantoide verwenden lässt, beispielsweise für Adamantan, Triamantan, Tetramantan, Pentamant, Hexamantan, Heptamantan, Octamantan, Nonamantan und Decamantan.

### Ausführungsbeispiele

### Ausführungsbeispiel 1: Herstellung von 1-Nitroxydiamantan

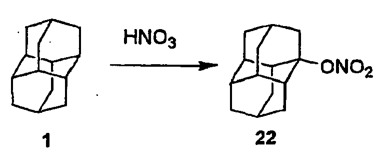

Diamantan 1 (19,3 g; 0,103 mol), CH₂Cl₂ (100 mal) und 99%-ige HNO₃ (38,5 mL; 0,91 moll werden bei 0 °C gemischt, 30 Minuten bei 20 °C gerührt, auf 0°C abgekühlt und der Säureüberschuss mit festem NaHCO₃ neutralisiert. Der Niederschlag wird abfiltriert und CH₂Cl₂ bei vermindertem Druck entfernt. Der nach Entfernen des CH₂Cl₂ verbleibende Rückstand wird aus n-Hexan umkristallisiert und ergibt 21,7 g (85%) 1-Nitroxydiamantan **22.**
¹H-NMR: (400 MHz, δ, ppm, CDCl₃): 2.30 bs (2H), 2.15 bs (3H), 2.05 m (6H), 1.68 m (4H), 1.60 m (2H), 1.47 m (2H).
¹³C-NMR: (100 MHz, δ, ppm, CDCl₃): 93.7 (C), 40.5 (CH), 39.3 (CH₂), 39.2 (CH), 37.5 (CH₂), 36.7 (CH₂), 36.3 (CH), 32.3 (CH₂), 30.3 (CH), 24.6 (CH). Charakteristische Banden im IR-Spektruni: (v, com⁻¹, CHCl₃): 2997 (C-H), 2855 (C-H), 1611 (N=O), 1306 (N=O), 1247 (C-0).

### Ausführungsbeispiel 2: Herstellung von 1-Acetaminodiamantan

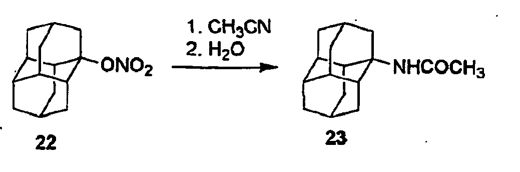

1 g (4.0 mmol) 1-Nitroxydiamantan **22** werden in 100 mL Acetonitril gelöst. Das Reaktionsgemisch wird für 10 h zum Rückfluss erhitzt und anschließend auf Raumtemperatur abgekühlt. Dann wird 1 mL Wasser hinzugefügt. Nach Entfernen der flüchtigen Bestandteile bei vermindertem Druck wird der Rückstand aus CCl₄ umkristallisiert. Es resultieren 0.78 g (79 %) 1 Acetaminodiarnantan **23.**
¹H-NMR: (400 MHz, δ, ppm, CDCl₃): 5.50 (brs, 1H), 2.22 (m, 2H), 2.03 (m, 2H), 1.93 (m, 4H), 1.90 (s, 4H), 1.49 (m, 2H), 1.45 (m, 8H).
¹³C-NMR: (100 MHz, δ, ppm, CDCl₃): 169.1. 56.1, 41.5, 39.1, 38.7, 38.1, 37.3, 37.0, 32.8, 28.6, 25.1, 24.5.
MS (70 eV) (m/z, %): 245 (71), 230 (14), 202 (7), 186 (94), 171 (4), 157 (6), 143 (11), 130 (45), 129 (41), 95 (90), 94 (100), 79 (25), 67 (18), 53 (11).

### Ausführungsbeispiel 3: Herstellung von 1-Diamantancarbonsäure

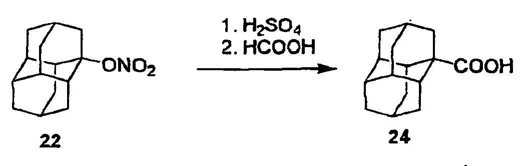

1 g (4,0 mmol) 1-Nitroxydiamantan **22** werden bei einer Temperatur von 10 °C unter Rühren zu einem Gemisch aus 25 mL 98 %-iger H₂SO₄ und 2 mL Ameisensäure gegeben. Zusätzlich werden bei dieser Temperatur innerhalb von 1 h weitere 3 mL Ameisensäure zugegeben. Das Reaktionsgemisch wird auf 100 g Eis gegeben und dreimal mit je 20 mL Chloroform extrahiert. Die vereinigten Extrakte werden zunächst mit Wasser, dann mit Sole gewaschen und über Na₂SO₄ getrocknet. Entfernen des Lösungsmittels bei vermindertem Druck und anschließende Umkristallisation des Rückstands aus CHCl₃ ergeben 0.75 g (81 %) 1-Diamantancarbonsäure **24.**

### Ausführungsbeispiel 4: Herstellung von 1-Acetoxydiamantan

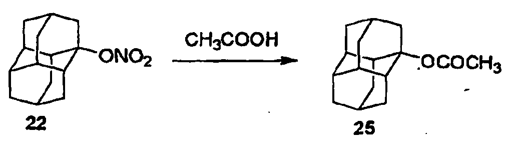

1 g (4,0 mmol) 1-Nitroxydiamantan **22** werden in 10 mL Essigsäure gelöst Das Reaktionsgemisch wird für 10 h bei 70 °C gerührt, a uf Raumtemperatur abgekühlt, mit 30 mL Wasser verdünnt und dreimal mit je 10 mL Chloroform extrahiert. Die vereinigten Extrakte werden zunächst mit Wasser, dann mit Sole gewaschen und über Na₂SO₄ getrocknet. Entfernen des Lösungsmittels bei vermindertem Druck und Umkristallisation des Rückstands aus n-Hexan ergeben 0,88 g (89 %) 1-Acetoxydiamantan **25.**

### Ausführungsbeisipiel 5: Herstellung von 1-Hvdroxydiamantan

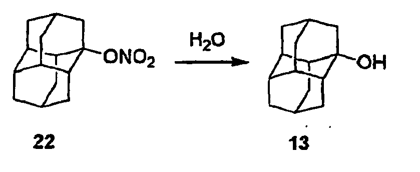

1 g (4,0 mmol) 1-Nitroxydiamantan **22** werden in 4 mL 30 %-iger Salpetersäure gelöst und für 3 h zum Rückfluss erhitzt. Das Reaktionsgemisch wird abgekühlt und filtiert Der Niederschlag wird im Vakuum getrocknet und aus Cyclohexan umkristallisiert. Es resultieren 0,73 g (90 %) 1-Hydroxydiamantan **13.**

### Ausführungsbeispiel 6: Herstellung von Dinitroxydiamantanen

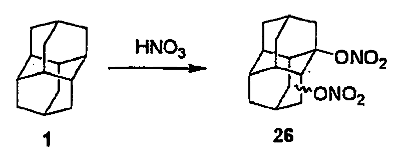

Diamantan **1** (10 g, 0,053 mol), CH₂Cl₂ (130 mL) und 99 %-ige HNO₃ (57 mL, 1,35 mol) werden bei 0 °C gemischt, für 10 h bei 25 °C g erührt, auf 0 °C abgekühlt und der Säureüberschuss mit trockenem NaHCO₃ neutralisiert. Der Niederschlag wird abfiltriert und CH₂Cl₂ bei vermindertem Druck entfernt. Der nach dem Entfernen des CH₂Cl₂ verbleibende Rückstand enthält ein Gemisch von Dinitroxydiamantanen **26** (16,1 g; 97 %).
¹H-NMR: (400 MHz, δ, ppm, CDCl₃): 2.40-2.00 m, 1.91-1:35 m. Charakteristische Signale der C-ONO₂-Gruppe im ¹³C-NMR: (100 MHz, δ, ppm, CDCl₃): 93.7 (C), 91.9 (C), 90.5 (C), 87.9 (C).
Charakteristische Banden im IR-Spektrum (v, cm⁻¹, CHCl₃): 2990 (C-H), 2860 (C-H), 1610 (N=O), 1306 (N=0), 1250 (C-0), 1245 (C-0).

### Ausführungsbeispiel 7: Umlagerung und Hydrolyse von Dinitroxydiamantanen

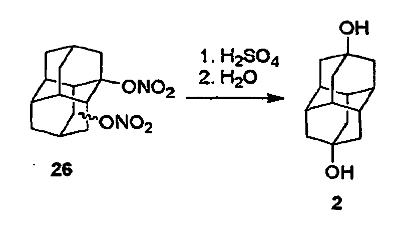

5 g des Gemisches der Dinitroxydiamantane **26** werden bei 0 °C zu 100 mL einer 98 %-igen H₂SO₄ gegeben und anschließend für 2,5 h bei Raumtemperatur gerührt Danach wird das Reaktionsgemisch auf 300 g Eis gegossen. Extraktion mit Chloroform, Entfernen des Lösungsmittels unter vermindertem Druck und Umkristallisation des Rückstandes aus Methanol ergeben 2,5 g (71 %) 4,9-Dihydroxydiamantan 2 in Form eines farblosen Feststoffes, Schmelzpunkt 291-293 °C (Lit. 290-292 °C).

### Ausführungsbeispiel 8: Hydrolyse und Umlagerung von Dinitroxydiamantanen

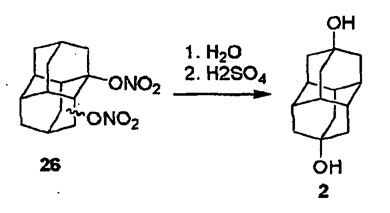

Diamantan **1** (1,28 g, 0,149 mol), CH₂Cl₂ (250 mL) und 100% HNO₃ (90 mL, 2,13 mol) werden bei 0 °C gemischt, für 2 h bei 25 °C ge rührt und im Anschluss mit Wasser (180 mL) verdünnt. Das CH₂Cl₂ wird entfernt, und die Reaktionsmischung **26** wird für 1,5 h refluziert. Entfernung des Wassers im Vakuum ergab als Rückstand eine Mischung verschiedener Oligohydroxyde **20.** Diese **20** werden zu 200 mL 96% H₂SO₄ gegeben und für 2 h bei Raumtemperatur gerührt. Danach wird die Reaktionsmischung auf 300 g Eis gegeben und filtriert. Extraktion des Filtrats mit einem oder mehreren organischen Lösemitteln (typischerweise Diethylether) ergibt 21,3 g (0,097 mmol, 65%) 4,9-Dihydroxydiamantan (2, m=0) als weißen Feststoff, Smp. 291-293 °C (aus Methanol, lit. 290-292 °C).

## Patentansprüche

1. Verbindung der Formel 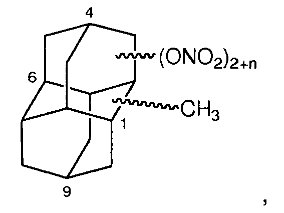
wobei n eine ganze Zahl zwischen 0 und 6 darstellt,
m eine ganze Zahl zwischen 0 und 1 darstellt,
die Methylgruppe im Falle von m = 1 bevorzugt an Position 1, 3 oder 4 des Diamantans gebunden ist,
und mindestens eine Nitroxygruppe an ein apicales Kohlenstoffatom gebunden ist.

2. Verfahren zur Herstellung eines mindestens zweifach nitroxylierten Diamantans, **gekennzeichnet durch** die Schritte
a) Mischen des zu nitroxylierenden Diamantans mit einem Nitroxylierungsreagenz bei -5 °C bis 5 °C im Stoffmengenverhältnis Diamantan : Nitroxylierungsmittel = 1 : 8 bis 1: 25 pro einzuführender Nitroxygruppe,
b) Rühren bei 20 °C bis 30 °C für 1 h bis 3 h.

3. Verfahren gemäß Anspruch 2, **dadurch gekennzeichnet, dass** als Nitroxylierungsmittel Nitriersäure oder konzentrierte Salpetersäure verwendet wird.

4. Verfahren gemäß Anspruch 2, **dadurch gekennzeichnet, dass** als Nitroxylierungsmittel eine Vorstufe der konzentrierten Salpetersäure verwendet wird, bevorzugt ein Gemisch aus konzentrierter Schwefelsäure und einem Alkalimetallnitrat oder ein Gemisch aus Ozon und Stickstoffdioxid, NO⁺X⁻ und NO₂⁺X⁻ Reagenzien, wobei X ein Halogen ist, ausgewählt aus Fluor, Chlor, Brom, Iod.

5. Verfahren gemäß einem der Ansprüche 2 bis 4, **dadurch gekennzeichnet, dass** in Schritt a) zusätzlich ein organisches Lösungsmittel verwendet wird, ausgewählt aus der Gruppe CH₂Cl₂, CHCl₃, CCl₄, Diethylether, THF, Ethylacetat oder ein Gemisch davon.

6. Verfahren zur Herstellung mindestens zweifach substituierter Diamantane, **gekennzeichnet durch** die Schritte
a) Herstellung eines mindestens zweifach nitroxylierten Diamantans gemäß einem der Ansprüche 2 bis 5,
b) Umsetzung des mindestens zweifach nitroxylierten Diamantans mit einem Nucleophil.

7. Verfahren zur Herstellung mindestens zweifach hydroxylierter Diamantane, **gekennzeichnet durch** die Schritte
a) Herstellung eines mindestens zweifach nitroxylierten Diamantans gemäß einem der Ansprüche 2 bis 5,
b) Zugabe von Wasser,
c) Erhitzen zum Rückfluss für 1 h bis 2 h,
d) Zugabe von 1,5 bis 5 Mol einer starken konzentrierten Säure pro Nitroxygruppe, die gemäß Schritt a) eingeführt wurde,
e) Rühren für 1 h bis 3 h bei 20 °C bis 30 °C,
f) Zugabe von je 1 Volumenteil der Reaktionsmischung aus Schritt e) zu 1 bis 2 Volumenteilen Eis.

8. Verfahren zur Herstellung mindestens zweifach hydroxylierter Diamantane, **gekennzeichnet durch** die Schritte
a) Herstellung eines mindestens zweifach nitroxylierten Diamantans gemäß einem der Ansprüche 2 bis 5,
b) Zugabe von 1,5 Mol bis 5 Mol einer starken konzentrierten Säure pro Nitroxygruppe, die gemäß Schritt a) eingeführt wurde,
c) Rühren bei 20 °C bis 30 °C für 1 h bis 3 h,
d) Zugabe der Reaktionsmischung aus Schritt c) zu 1 bis 3 Volumenteilen Eis pro Volumenteil der Reaktionsmischung.

9. Verfahren zur Herstellung mindestens zweifach hydroxylierter Diamantane gemäß einem der Ansprüche 7 und 8, **dadurch gekennzeichnet, dass** die starke konzentrierte Säure in Schritt e) ausgewählt ist aus der Gruppe Schwefelsäure, Salzsäure, Bromwasserstoffsäure, lodwasserstoffsäure, Salpetersäure, Phosphorsäure.

10. Verfahren zur Herstellung mindestens zweifach substituierter Diamantane, **gekennzeichnet durch** die Schritte
a) Herstellung eines mindestens zweifach nitroxylierten Diamantans gemäß einem der Ansprüche 2 bis 5,
b) Umsetzen des mindestens zweifach nitroxylierten Diamantans mit einem Nucleophil, ausgewählt aus der Gruppe HCOOH, CH₃COOH, HCONH₂, CH₃CN.

11. Verfahren zur Herstellung mindestens zweifach substituierter Diamantane, **gekennzeichnet durch** die Schritte
c) Herstellung eines mindestens zweifach nitroxylierten Diamantans gemäß einem der Ansprüche 2 bis 5,
d) Zugabe von 1,5 Mol bis 5 Mol einer starken konzentrierten Säure pro Nitroxygruppe, die gemäß Schritt a) eingeführt wurde,
e) Rühren bei 20 °C bis 30 °C für 1 h bis 3 h,
f) Umsetzen des Reaktionsgemisches aus Schritt c) mit einem Nucleophil, ausgewählt aus der Gruppe HCOOH, CH₃COOH, HCONH₂, CH₃CN.

12. Verfahren zur Herstellung mindestens zweifach substituierter Diamantane, **gekennzeichnet durch** die Schritte
a) Herstellung eines mindestens zweifach hydroxylierten Diamantans gemäß einem der Ansprüche 7 bis 9,
b) Umsetzen des zweifach hydroxylierten Diamantans mit einem Nucleophil, ausgewählt aus der Gruppe HCOOH, CH₃COOH, HCONH₂, CH₃CN.

13. Verfahren zur Herstellung substituierter Diamantane gemäß einem der Ansprüche 7 bis 12, **dadurch gekennzeichnet, dass** zusätzlich und als letzter Schritt eine Reinigung des Produktes durchgeführt wird.

## Claims

1. Compound of the formula 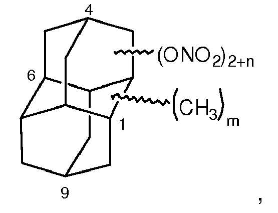
wherein n represents an integer between 0 and 6,
m represents an integer between 0 and 1,
the methyl group in the case of m = 1 is bound preferably to position 1, 3 or 4 of the diamantane,
and at least one nitroxy group is bound to an apical carbon atom.

2. Method for producing an at least dinitroxylated diamantane, **characterised by** the steps
a) mixture of the diamantane to be nitroxylated with a nitroxylating agent at -5°C to 5°C in the molar ratio diamantane : nitroxylating agent = 1 : 8 to 1 : 25 per nitroxy group to be introduced,
b) stirring at 20°C to 30°C for 1h to 3h.

3. Method according to claim 2, wherein sulphonitric acid or concentrated nitric acid is used as nitroxylating agent.

4. Method according to claim 2, wherein a precursor of the concentrated nitric acid is used as nitroxylating agent, preferably a mixture of concentrated sulphuric acid and an alkali metal nitrate
or a mixture of ozone and nitrogen dioxide, NO⁺X⁻ and NO₂⁺X- reagents, wherein X represents a halogen selected from fluorine, chlorine, bromine, iodine.

5. Method according to one of the claims 2 to 4, wherein in step a) is used additionally an organic solvent selected from the group CH₂Cl₂, CHCl₃, CCl₄, diethylether, THF, ethyl acetate or a mixture thereof.

6. Method for producing at least disubstituted diamantanes, **characterised by** the steps
a) production of an at least dinitroxylated diamantane according to one of the claims 2 to 5,
b) reaction of the at least dinitroxylated diamantane with a nucleophile.

7. Method for producing at least dihydroxylated diamantanes, **characterised by** the steps
a) production of an at least dinitroxylated diamantane according to one of the claims 2 to 5,
b) addition of water,
c) heating to reflux for 1 h to 2h,
d) addition of 1.5 to 5 mol of a strong concentrated acid per nitroxy group which had been introduced according to step a),
e) stirring for 1 h to 3h at 20°C to 30°C,
f) addition of 1 part by volume of the reaction mixture, respectively, from step e) to 1 to 2 parts by volume of ice.

8. Method for producing at least dihydroxylated diamantanes, **characterised by** the steps
a) production of an at least dinitroxylated diamantane according to one of the claims 2 to 5,
b) addition of 1.5 mol to 5 mol of a strong concentrated acid per nitroxy group which had been introduced according to step a),
c) stirring at 20°C to 30°C for 1 h to 3h,
d) addition of the reaction mixture from step c) to 1 to 3 parts by volume ice for every part of volume of the reaction mixture.

9. Method for producing at least dihydroxylated diamantanes according to one of the claims 7 and 8, wherein the strong concentrated acid is selected from the group sulphuric acid, hydrochloric acid, hydrobromic acid, hydroiodic acid, nitric acid, phosphoric acid.

10. Method for producing at least disubstituted diamantanes, **characterised by** the steps
a) production of an at least dinitroxylated diamantane according to one of the claims 2 to 5,
b) reaction of the at least disubstituted diamantane with a nucleophile selected from the group HCOOH, CH₃COOH, HCONH₂, CH₃CN.

11. Method for producing at least disubstituted diamantanes, **characterised by** the steps
c) production of an at least dinitroxylated diamantane according to one of the claims 2 to 5,
d) addition of 1.5 mol to 5 mol of a strong concentrated acid per nitroxy group which had been introduced according to step a),
e) stirring at 20°C to 30°C for 1 h to 3h,
f) reaction of the reaction mixture from step c) with a nucleophile selected from the group HCOOH, CH₃COOH, HCONH₂, CH₃CN.

12. Method for producing at least disubstituted diamantanes, **characterised by** the steps
a) production of an at least dinitroxylated diamantane according to one of the claims 7 to 9,
b) reaction of the dihydroxylated diamantane with a nucleophile selected from the group HCOOH, CH₃COOH, HCONH₂, CH₃CN.

13. Method for producing substituted diamantanes according to one of the claims 7 to 12, wherein additionally, and as the final step, purification of the product is carried out.

## Revendications

1. Composé de la formule 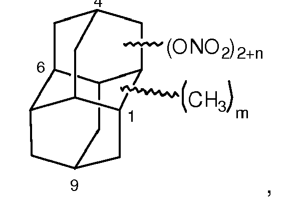
où n représente un nombre entier entre 0 et 6,
m représente un nombre entier entre 0 et 1,
le groupe méthyle est lié, en cas de m = 1, de façon préférée à la position1, 3 ou 4 du diamantane,
et au moins un groupe nitroxy est lié à un atome de carbone apical.

2. Procédé pour la production d'un diamantane au moins dinitroxylé, **caractérisé par** les étapes
a) Mélanger le diamantane à nitroxyler avec un réactif de nitroxylation à -5 °C à 5 °C dans le rapport molaire diamantane : agent de nitroxylation = 1 : 8 à 1 : 25 par groupe nitroxyle à introduire,
b) agiter à 20 °C à 30 °C pour 1 h à 3 h.

3. Procédé selon la revendication 2, **caractérisé en ce que** de l'acide sulfonitrique ou de l'acide nitrique concentré est utilisé comme agent de nitroxylation.

4. Procédé selon la revendication 2, **caractérisé en ce qu'**un précurseur de l'acide nitrique concentré, de façon préférée un mélange d'acide sulfurique concentré et un nitrate de métal alcalin est utilisé comme agent de nitroxylation ou un mélange d'ozone et de dioxyde d'azote, de réactifs NO⁺X⁻ et NO₂⁺X⁻, où X est un halogène, sélectionné de fluor, chlore, brome, iode.

5. Procédé selon l'une des revendications 2 à 4, **caractérisé en ce que** dans l'étape a) un solvant organique est utilisé en plus, sélectionné du groupe CH₂Cl₂, CHCl₃, CCl₄, éther diéthylique, THF, acétate d'éthyle ou un mélange d'eux.

6. Procédé pour la production de diamantanes au moins disubstitués, **caractérisé par** les étapes
a) Production d'un diamantane au moins dinitroxylé selon l'une des revendications 2 à 5,
b) réaction du diamantane au moins dinitroxylé avec un nucléophile.

7. Procédé pour la production de diamantanes au moins dihydroxylés, **caractérisé par** les étapes
a) Production d'un diamantane au moins dinitroxylé selon l'une des revendications 2 à 5,
b) addition d'eau,
c) chauffage à reflux pour 1 h à 2 h,
d) addition de 1,5 à 5 moles d'un acide fort concentré par groupe nitroxyle, introduit selon l'étape a),
e) agitation pour 1 h à 3 h à 20 °C à 30 °C,
f) addition de respectivement 1 fraction volumique du mélange réactionnel de l'étape e) à 1 à 2 fractions volumiques de glace.

8. Procédé pour la production de diamantanes au moins dihydroxylés, **caractérisé par** les étapes
a) Production d'un diamantane au moins dinitroxylé selon l'une des revendications 2 à 5,
b) addition de 1,5 à 5 moles d'un acide fort concentré par groupe nitroxyle, introduit selon l'étape a),
c) agitation à 20 °C à 30 °C pour 1 h à 3 h,
d) addition du mélange réactionnel de l'étape c) à 1 à 3 fractions volumiques de glace par fraction volumique du mélange réactionnel.

9. Procédé pour la production de diamantanes au moins dihydroxylés, selon l'une des revendications 7 et 8, **caractérisé en ce que** l'acide fort concentré est sélectionné du groupe acide sulfurique, acide chlorhydrique, acide bromhydrique, acide iodhydrique, acide nitrique, acide phosphorique.

10. Procédé pour la production de diamantanes au moins doublement substitués, **caractérisé par** les étapes
a) Production d'un diamantane au moins dinitroxylé selon l'une des revendications 2 à 5,
b) réaction du diamantane au moins disubstitué avec un nucléophile, sélectionné du groupe HCOOH, CH₃COOH, HCONH₂, CH₃CN.

11. Procédé pour la production de diamantanes au moins doublement substitués, **caractérisé par** les étapes
c) Production d'un diamantane au moins dinitroxylé selon l'une des revendications 2 à 5,
d) addition de 1,5 à 5 moles d'un acide fort concentré par groupe nitroxyole, introduit selon l'étape a),
e) agitation à 20 °C à 30 °C pour 1 h à 3 h,
f) réaction du mélange réactionnel de l'étape c) avec un nucléophile, sélectionné du groupe HCOOH, CH₃COOH, HCONH₂, CH₃CN.

12. Procédé pour la production de diamantanes au moins disubstitués, **caractérisé par** les étapes
a) Production d'un diamantane au moins dinitroxylé selon l'une des revendications 7 à 9,
b) réaction du diamantane dihydroxylé avec un nucléophile, sélectionné du groupe HCOOH, CH₃COOH, HCONH₂, CH₃CN.

13. Procédé pour la production de diamantanes substitués, selon l'une des revendications 7 à 12, **caractérisé en ce qu'**en plus et comme dernière étape un nettoyage du produit est effectué.
